# EUROPÄISCHE PATENTSCHRIFT

(11) **EP 2 640 694 B1**
(45) Veröffentlichungstag und Bekanntmachung des Hinweises auf die Patenterteilung: **09.01.2019**
(21) Anmeldenummer: 11782610.7
(22) Anmeldetag: 14.11.2011
(51) Int. Cl.: C07C 263/10, C07C 265/14

(54) **VERFAHREN ZUR HERSTELLUNG VON METHYLENDIPHENYLDIISOCYANAT**
PROCESS FOR THE PRODUCTION OF METHYLENEDIPHENYLDIISOCYANATE
PROCÉDÉ DE PRODUCTION DE DIISOCYANATE DE MÉTHYLÈNEDIPHÉNYLE

(30) Priorität: 17.11.2010 EP 10191557
(43) Veröffentlichungstag der Anmeldung: 25.09.2013
(73) Patentinhaber: BASF SE, 67056 Ludwigshafen am Rhein (DE)
(72) Erfinder: BOCK, Michael, 67152 Ruppertsberg (DE); THIELE, Kai, B-2040 Antwerpen 4 (BE); SCHELLING, Heiner, 67281 Kirchheim (DE)
(74) Vertreter: BASF IP Association
(86) Internationale Anmeldenummer: PCT/EP2011/069993
(87) Internationale Veröffentlichungsnummer: WO 2012/065927

(56) Entgegenhaltungen:
- EP-A1- 1 506 957
- EP-A1- 1 792 895
- EP-A2- 0 116 847
- EP-A2- 1 561 746

## Beschreibung

Die Erfindung betrifft ein Verfahren zur Herstellung von Methylendiphenyldiisocyanat umfassend (a) die Phosgenierung von zwei und/oder mehrkernigem Methylendiphenyldiamin, (b) die Auftrennung des erhaltenen rohen Methylendiphenyldiisocyanat in zwei- und mehrkernige Isomere, (c) die Aufreinigung und/oder Auftrennung der erhaltenen Mischung zweikerniger Isomere des Methylendiphenyldiisocyanat, und (d) die Lagerung der in Schritt (c) beziehungsweise in Schritt (b) erhaltenen Mischungen, wobei ein Teil der gelagerten Mischungen mittels Rückführung wieder in mindestens einem der Schritte (b) oder (c) eingesetzt wird.

Methylendiphenyldiisocyanat (MDI) ist ein wichtiges Ausgangsprodukt für die Herstellung von Polyurethanen und verwandten Polymeren, die beispielsweise in Schäumen und Beschichtungen Verwendung finden.

Die säurekatalysierte Herstellung von Methylendiphenyldiisocyanat (MDI) ausgehend von Anilin und Formaldehyd ist bekannt und führt zunächst zu einem komplexen Gemisch aus Polyaminen, das anschließend mit Phosgen umgesetzt wird. Hierbei wird zunächst ein komplexes Gemisch aus zwei- und mehrkernigem MDI erhalten, welches nachfolgend als rohes Methylendiphenyldiisocyanat (Roh-MDI) bezeichnet wird. Roh-MDI besteht insbesondere aus den zweikernigen Isomeren 4,4'-MDI, 2,4'-MDI und in geringerem Maße 2,2'-MDI (zusammen nachfolgend als rohes zweikerniges MDI bezeichnet) sowie aus drei- oder mehrkernigem MDI, welches nachfolgend als polymeres MDI bezeichnet wird (PMDI).

In bekannten Verfahren wird rohes MDI in eine PMDI-reiche Mischung und in rohes zweikerniges MDI aufgetrennt. Anschließend erfolgt üblicherweise die Abtrennung von 4,4'-MDI einerseits und einer 2,4'-MDI-reichen Mischung andererseits aus dem rohen zweikernigen MDI. Entsprechende Verfahren werden beispielsweise in den Offenlegungsschriften DE 1923214, DE 102005004170, DE 102005055189, CN 101003497 und DE 10333929 beschrieben. EP-A-0116847 offenbart einen Prozess für die Verwendung von Sedimenten, die sich während der Lagerung von reinem MDI gebildet haben.

Vor der weiteren Verarbeitung müssen die so hergestellten, in flüssiger Form vorliegenden Methylendiphenyldiisocyanat-Produkte temporär aufbewahrt und/oder gelagert werden.
Methylendiphenyldiisocyanat, insbesondere zweikerniges MDI, bildet in flüssiger Phase nach einiger Zeit, d. h. während der Lagerung, dimere Folgeprodukte. Hierbei spielt insbesondere die Bildung von Uretdionen durch 4-Ringbildung infolge Dimerisierung zweier Isocynatgruppen und die Bildung von Uretoniminen durch 4-Ringbildung aus einer Carbodiimidgruppe und einer Isocyanatgruppe eine wichtige Rolle. Die Bildung der 4-Ringe ist grundsätzlich eine Gleichgewichtsreaktion, die durch Temperaturerhöhung auf die Seite der Isocyanate bzw. Carbodiimide verschoben werden kann. Die Bildung von Uretdionen erfolgt bei aromatischen Isocyanaten auch unkatalysiert. Eine Trimerisierung zu den sogenannten Isocyanuraten (1,3,5-Triazin-2,4,6-trione) ist ebenfalls möglich, verläuft jedoch im Allgemeinen nur bei Zugabe eines geeigneten Katalysators in nennenswerter Geschwindigkeit.

Die Bildung der in dem Methylendiphenyldiisocyanat unlöslichen dimeren Folgeprodukte führt zu nachteiligen Trübungen und Sedimentationen und resultiert in Qualitätsminderungen in der nachfolgenden Weiterverarbeitung, insbesondere durch Verstopfung von Leitungen, Apparaten und Maschinen. Demzufolge ist es wünschenswert, ein Verfahren zur Herstellung von Methylendiphenyldiisocyanat bereitzustellen, welches die vorgenannten Probleme vermeidet.

Verfahren zur Rückspaltung von Sedimenten in Methylendiphenyldiisocyanat sind an sich bekannt. Die EP 116847 A2 beschreibt die Herstellung von rohem Methylendiphenyldiisocyanat aus Lagersedimenten. Das dort beschriebene Sediment besteht aus dimerem Methylendiphenyldiisocyanat (Uretdion) und polymeren MDI-Dimeren. Die Sedimente werden auf 180°C bis 250°C in Gegenwart von rohem MDI 0,25 bis 6 Stunden getempert und anschließend auf eine Temperatur von 60°C oder weniger in 5 Minuten abgekühlt. Die so rückgespaltenen Sedimente können als rohes Methylendiphenyldiisocyanat für die Herstellung von Polyurethanprodukten verwendet werden. Ein solches, im Anschluss an die Herstellung von zweikernigem Methylendiphenyldiisocyanat durchgeführtes Verfahren ist jedoch aufwendig, beispielsweise indem es einen Abtrennungsschritt und zusätzliche Apparaturen erfordert. Ein Verfahren, welches die Bildung von Sedimenten ohne derartige separate Rückspaltungsverfahren verringert, wäre somit wünschenswert.

Es war daher Aufgabe der Erfindung ein Verfahren zur Herstellung von Methylendiphenyldiisocyanat aufzufinden, das die vorgenannten Nachteile nicht oder in geringerem Umfang aufweist.

Aufgabe der vorliegenden Erfindung war es, MDI, insbesondere Mischungen aus 2,4'- und 4,4'-MDI sowie reines 4,4'-MDI, mit einem geringen Gehalt an Uretdionen und Uretoniminen herzustellen. Das Verfahren sollte mit einem geringen apparativen Aufwand zu realisieren sein und gegenüber MDI schonend sein. Das Verfahren sollte mit möglichst geringem verfahrenstechnischem Aufwand in bestehende Technologien zur Herstellung von zweikernigem MDI zu integrieren sein. Das Verfahren sollte die Bildung von Sedimenten und Ablagerungen während der Lagerung von MDI reduzieren oder verhindern sowie den Zeitraum der sediment- und ablagerungsfreien Lagerung gegenüber dem Stand der Technik erhöhen.

Die vorgenannten Aufgaben werden gelöst durch das erfindungsgemäße Verfahren. Bevorzugte Ausführungsformen sind den Ansprüchen und der nachfolgenden Beschreibung zu entnehmen. Kombinationen bevorzugter Ausführungsformen einzelner Verfahrensschritte verlassen den Rahmen der vorliegenden Erfindung nicht.

Das erfindungsgemäße Verfahren zur Herstellung von Methylendiphenyldiisocyanat umfasst folgende Schritte:
(a) Phosgenierung von zwei und/oder mehrkernigem Methylendiphenyldiamin unter Erhalt von zwei und/oder mehrkernigem Methylendiphenyldiisocyanat,
(b) Auftrennung des rohen Methylendiphenyldiisocyanat aus Schritt (a) unter Erhalt von mindestens einer Mischung M enthaltend zweikernige Isomere des Methylendiphenyldiisocyanat und mindestens einer Mischung P enthaltend oligomeres Methylendiphenyldiisocyanat,
(c) Aufreinigung und/oder Auftrennung mindestens einer Mischung M unter Erhalt von mindestens einer weiteren Mischung F enthaltend mindestens eine Verbindung ausgewählt aus 2,2'-Methylendiphenyldiisocyanat, 2,4'-Methylendiphenyldiisocyanat und 4,4'-Methylendiphenyldiisocyanat, und
(d) Lagerung der in Schritt (c) erhaltenen Mischung F oder Mischungen F sowie der in Schritt (b) erhaltenen Mischung P für einen Zeitraum von mindestens 1 Tag bei einer Temperatur von 5°C bis 60°C,
dadurch gekennzeichnet, dass ein Teil mindestens einer der gelagerten Mischungen F und/oder P mittels Rückführung wieder in mindestens einem der Schritte (b) oder (c) eingesetzt wird, wobei die Rückführung der gelagerten Mischungen F und/oder P an eine Stelle erfolgt, an der Temperatur und Verweilzeit dergestalt sind, dass die Rückspaltung von Uretdioneinheiten in Isocyanateinheiten erfolgt.

Der Begriff "Auftrennung" kennzeichnet die teilweise oder vollständige Trennung eines Stoffgemisches in Einzelbestandteile oder Mischungsbestandteile.

Der Begriff "Aufreinigung" kennzeichnet die Reduktion des Gehaltes an Nebenkomponenten in einem Gemisch und somit die Erhöhung des Reinheitsgrades eines Gemisches in Bezug auf eine bestimmte Komponente.

"Lagerung" ist die temporäre Aufbewahrung eines Stoffes oder Stoffgemisches zwecks späterer Verwendung und/oder Entnahme. "Lagerung" bedeutet insbesondere die temporäre Aufbewahrung eines Stoffes oder Stoffgemisches für einen Zeitraum von mindestens 1 Tag.

Der Begriff "Rückführung" kennzeichnet die Zuführung einer bestimmten Menge eines Stoffes aus einer Vorrichtung zur Lagerung in eine vorhergehende Stufe des Verfahrens zur Herstellung von MDI.

Der Begriff "Destillation" beziehungsweise "destillativ" kennzeichnet die Aufreinigung eines Gemisches mittels eines destillativen Trennverfahrens. Destillative Trennerfahren sind dadurch gekennzeichnet, dass die Trennwirkung auf der unterschiedlichen Zusammensetzung der siedenden Flüssigkeit und des gasförmigen Dampfes beruht.

Der Gehalt in ppm bezieht sich im Rahmen der vorliegenden Erfindung grundsätzlich auf Gewichtsanteile bezogen auf das Gesamtgewicht eines Gemisches.

Eine Kolonne ist eine Vorrichtung zur destillativen Aufreinigung eines Gemisches. Im Rahmen der vorliegenden Erfindung wird unter Kolonne eine Rektifikationskolonne verstanden. Kolonnen sind dem Fachmann an sich bekannt.

Eine Kolonne umfasst einen vorzugsweise längsförmigen Behälter mit Trennelementen. Trennelemente sind Einbauten, die die Wärme- und Stoffübertragung intensivieren. Die Kolonne umfasst zudem einen Bereich unterhalb des untersten Trennelementes, der Kondensat aufnehmen kann (den Sumpf) und einen Bereich oberhalb des obersten Trennelementes, den Kopf. Zwecks Verdampfung des zu trennenden Stoffgemisches kann unterhalb des Sumpfes der Rektifikationskolonne ein Verdampfer angeordnet sein. Zwecks Kondensation des am Kopf austretenden Gasstromes kann hinter dem Kolonnenkopf ein Kondensator angeschlossen sein.

Je nach Art der verwendeten Trennelemente wird unterschieden zwischen Boden-, Füllkörper- und Packungskolonnen. Durch einen Zulauf, der häufig am Boden einer Kolonne angebracht ist, wird das verdampfte Gemisch der zu trennenden Stoffe eingespeist. Zum Kopf hin reichert sich die leichter siedende Komponente an und kann dort abgezogen werden, während die schwerer siedende Komponente zurückgeführt wird. Im Sumpf reichert sich die schwerer siedende Komponente an und kann dort abgenommen werden.

Man unterscheidet üblicherweise drei Typen von Trennelementen. In Bodenkolonnen sind Sieb -, Glocken- oder Ventilböden eingebaut, auf denen die Flüssigkeit steht. Durch spezielle Schlitze oder Löcher wird der Dampf in die Flüssigkeit eingeperlt, so dass eine Sprudelschicht entsteht. Auf jedem dieser Böden stellt sich ein neues temperaturabhängiges Gleichgewicht zwischen der Flüssig- und Gasphase ein.

Füllkörperkolonnen können mit unterschiedlichen Füllkörpern gefüllt werden, die eine gute Verteilung der Flüssigkeit und Verwirbelung der Gasströmung bewirken. Durch die Oberflächenvergrößerung werden Wärme- und Stoffaustausch optimiert und die Trennfähigkeit der Kolonne somit erhöht. Bekannte Beispiele sind der Raschig-Ring (ein Hohlzylinder), Pall-Ring, Hiflow-Ring, Intalox-Sattel, Berl-Sattel und Igel. Die Füllkörper können geordnet, aber auch regellos (als Schüttung) in die Kolonne eingebracht werden.

Packungskolonnen mit Packungen als Trennkörper (Packungselemente) sind eine Weiterentwicklung der geordneten Füllkörper. Sie weisen eine regelmäßig geformte Struktur auf. Dadurch ist es bei Packungen möglich, Einengungen für die Gasströmung (mit erheblichem Einfluss auf den Druckverlust) zu reduzieren. Es gibt verschiedene Ausführungen von Packungen, z. B. Gewebe- oder Blechpackungen.

Die Kondensation von Anilin und Formaldehyd unter Bildung von rohem Methylendiphenyldiamin (rohes MDA) ist aus dem Stand der Technik hinreichend bekannt. Rohes Methylendiphenyldiamin enthält zwei und/oder mehrkerniges Methylendiphenyldiamin.

Die technische Herstellung von rohem Methylendiphenyldiamin erfolgt im Allgemeinen in zwei Stufen. In einer ersten Stufe wird zunächst eine säurekatalysierte Kondensation von Anilin mit Formaldehyd zur Erzeugung des entsprechenden MDA-Gemisches durchgeführt. Dabei wird als Säurekatalysator üblicherweise eine starke Mineralsäure wie wässrige Salzsäure eingesetzt. Verfahren zur Herstellung von MDA durch säurekatalysierte Anilin-Formaldehydkondensation sind dem Fachmann hinlänglich bekannt und werden beispielsweise in der WO 99/40059 beschrieben.

Durch Auswahl der Mengenverhältnisse von Anilin, Formaldehyd und Mineralsäure sowie der Temperatur- und Verweilzeitbedingungen können die Anteile an 4,4'- Methylendiphenyldiamin sowie dessen Isomeren und Homologen gesteuert werden. Die Kondensation kann technisch sowohl kontinuierlich als auch diskontinuierlich durchgeführt werden. In einer zweiten Stufe wird vorzugsweise die erhaltene Reaktionslösung, vorteilhafterweise nach Neutralisation und Phasentrennung, destillativ von Wasser und Anilin befreit und zum rohen Methylendiphenyldiamin aufgearbeitet.

### Schritt (a)

Erfindungsgemäß erfolgt im Rahmen von Schritt (a) die Phosgenierung von zwei und/oder mehrkernigem Methylendiphenyldiamin unter Erhalt von zwei und/oder mehrkernigem Methylendiphenyldiisocyanat. Die Phosgenierung von MDA ist dem Fachmann weitläufig bekannt. Das eingesetzte MDA wird auch als rohes MDA, das hieraus resultierende MDI auch als rohes MDI bezeichnet.

Die Umsetzung kann in der Gasphase oder in der flüssigen Phase diskontinuierlich oder kontinuierlich durchgeführt werden, wie beispielsweise in W. Siefken, Liebig Annalen der Chemie 562, 75 (1949) beschrieben. Die kontinuierliche Herstellung von organischen Isocyanaten durch Reaktionen von primären organischen Aminen und Phosgen ist vielfach beschrieben und im großtechnischen Maßstab üblich und beispielsweise in Ullmanns Enzyklopädie der technischen Chemie, Band 7, dritte Auflage, Carl Hanser Verlag, S. 76 ff. (1993) beschrieben.

Heutige großtechnische Synthesen von Isocyanaten erfolgen fast ausschließlich in kontinuierlichen Verfahren. In der Regel ist die kontinuierliche Ausführungsform des Verfahrens mehrstufig. In der ersten Stufe der Phosgenierung wird das Amin mit Phosgen zum entsprechenden Carbamoylchlorid und Chlorwasserstoff und zu Aminhydrochloriden umgesetzt.

Die primäre Reaktion zwischen Aminen und Phosgen ist sehr schnell und exotherm. Zwecks Minimierung der Bildung von Nebenprodukten und Feststoffen werden Amin und Phosgen, beide gegebenenfalls in einem organischen Lösungsmittel gelöst, schnell vermischt. Die nächste Stufe der Phosgenierung umfasst sowohl die Zersetzung des Carbamoylchlorid zum gewünschten Isocyanat und Chlorwasserstoff als auch die Phosgenierung des Aminhydrochlorids zum Carbamoylchlorid. Flüssigphasenphosgenierungen sind zum Beispiel beschrieben in den Offenlegungsschriften EP 1 616 857, WO 2004/056756, WO 2006/130405 und EP 012 70 544. Zur Vermeidung der Bildung von den unerwünschten Zwischenprodukten der Aminhydrochloride kann die Phosgenierung auch als Gasphasenphosgenierung bei hohen Temperaturen geführt werden. Verfahren sind beispielhaft beschrieben in den Offenlegungsschriften EP 1 449 826 und WO 2008/006775 (Aerosolphosgenierung). Auch Phosgenierungen im überkritischen Bereich sind beschrieben worden (WO 2008/049783).

Im erfindungsgemäßen Verfahren kann die Phosgenierung beispielsweise unter Verwendung eines üblichen, bevorzugt inerten Lösungsmittels durchgeführt werden. Geeignete Lösungsmittel sind beispielsweise Monochlorbenzol, Dichlorbenzol oder sonstige chlorierte, aromatische Kohlenwasserstoffe wie Toluol oder Xylol. Bei der Phosgenierung werden vorzugsweise Temperaturen von 70 bis 120 °C und Drücke von 8 bis 5 bar eingestellt. Die Phosgenierung kann in einer oder mehreren Stufen durchgeführt werden. Beispielsweise kann die Phosgenierung durch eine zweistufige Umsetzung in Gegenwart mindestens eines inerten organischen Lösungsmittels durchgeführt werden, wobei die erste Stufe der Phosgenierung in einem statischen Mischer und die zweite Stufe der Phosgenierung in einem Verweilzeitapparat erfolgt.

Entsprechend dem eingesetzten MDA werden im erfindungsgemäßen Verfahren durch die Phosgenierung die entsprechenden MDI-Isomere 2,2'-, 2,4'- und/oder 4,4'-MDI sowie PMDI erhalten.

Nach der Phosgenierung des zwei und/oder mehrkernigen Methylendiphenyldiamin findet bevorzugt eine Aufarbeitung des Reaktionsmischung statt, bei der überschüssiges Phosgen und Lösungsmittel abgetrennt werden. In einer möglichen Ausführungsform kann sich auch noch eine physikalische, z. B. thermische und/oder eine chemische Nachbehandlung zur Entfernung störender Nebenprodukte anschließen. Entsprechende Verfahren sind dem Fachmann ebenfalls hinlänglich bekannt und sind beispielsweise in US 3912600, DD 288599, US 5364958, EP 0133538, JP 06345707, DD 288598, DD 288593, EP 0524507 und EP 0866057 beschrieben.

Aus Schritt a) erhält man so vorzugsweise ein Gemisch von zwei und/oder mehrkernigem MDI enthaltend 10 bis 90 Gew.-%, bevorzugt 20 bis 75 Gew.-%, besonders bevorzugt 30 bis 60 Gew.-% 4,4'-MDI, sowie 1 bis 12 Gew.-%, bevorzugt 2 bis 10 Gew.-%, besonders bevorzugt 4 bis 9 Gew.-% an 2,4'- und/oder 2,2'-MDI, und 9 bis 78 Gew.-%, bevorzugt 23 bis 70 Gew.-%, besonders bevorzugt 36 bis 61 Gew.-% an PMDI, jeweils bezogen auf das Gesamtgewicht des erhaltenen MDI-Gemisches.

### Schritt (b)

Erfindungsgemäß erfolgt anschließend im Rahmen von Schritt (b) die Auftrennung des rohen Methylendiphenyldiisocyanat unter Erhalt von mindestens einer Mischung M enthaltend zweikernige Isomere des Methylendiphenyldiisocyanat und mindestens einer Mischung P enthaltend polymeres Methylendiphenyldiisocyanat.

Die Mischung M wird auch als rohes zweikerniges Methylendiphenyldiisocyanat (MDI) bezeichnet. Rohes zweikerniges MDI ist dem Fachmann als Gemisch bekannt, welches neben 4,4'-Methylendiphenyldiisocyanat noch mindestens eines der Isomeren 2,2'- und 2,4'-Methylendiphenyldiisocyanat enthält. Die Mischung M enthält vorzugsweise von 80 bis 100 Gew.-% Gew.-%, insbesondere 85 bis 100 Gew.-% zweikerniges MDI, wobei das zweikernige MDI vorzugsweise aus 80 bis 100, insbesondere von 85 bis 95 Gew.-% 4,4'-MDI, von 0 bis 18 Gew.-%, insbesondere 5 bis 14 Gew.-% 2,4'-MDI und von 0 bis 2, insbesondere von 0 bis 1 Gew.-% 2,2'-MDI besteht, jeweils bezogen auf das Gesamtgewicht der Mischung M.

Die Mischung P enthaltend polymeres Methylendiphenyldiisocyanat (PMDI) enthält vorzugsweise von 40 bis 80 Gew.-%, insbesondere 45 bis 75 Gew.-% PMDI und von 20 bis 60 Gew.-%, insbesondere 25 bis 55 Gew.-% zweikerniges MDI.

Verfahren zur Auftrennung von rohem Methylendiphenyldiisocyanat sind dem Fachmann bekannt. Die Auftrennung kann über bekannte Verfahren wie beispielsweise Destillation, Lösungsmittelextraktion, Extraktion mit überkritischen Medien, wie z. B. überkritischem CO₂, oder Kristallisation erfolgen. Bevorzugt sind Destillation und/oder Kristallisation, die beispielsweise in den Offenlegungsschriften DE 1938384, DE 2631168 , EP 79516 und EP 1475367 beschrieben werden.

Im Rahmen von Schritt (b) erfolgt vorzugsweise eine destillative Auftrennung des rohen Methylendiphenyldiisocyanat unter Erhalt von mindestens einer Mischung M enthaltend zweikernige Isomere des Methylendiphenyldiisocyanat und mindestens einer Mischung P enthaltend oligomeres Methylendiphenyldiisocyanat. Die destillative Auftrennung kann grundsätzlich in verschiedenen Vorrichtungen erfolgen. Die destillative Auftrennung erfolgt vorzugsweise in einem Umlaufverdampfer, insbesondere Fallfilmverdampfer oder Zwangsumlaufverdampfer, oder in Aufkochern, insbesondere solche vom Kettle-Typ. Bei hohem Aufkonzentrierungsgrad kann die destillative Auftrennung auch in Dünnschichtverdampfern oder Wendelrohrverdampfern durchgeführt werden. Die Auftrennung kann einstufig oder mehrstufig ausgeführt werden. Bei der mehrstufigen Ausführung sind die vorgenannten Verdampfer vorzugsweise Sumpfverdampfer einer Destillationskolonne.

In einer besonders bevorzugten Ausführungsform erfolgt im Rahmen von Schritt (b) die Auftrennung mittels eines Fallfilmverdampfers.

### Schritt (c)

Erfindungsgemäß erfolgt im Rahmen von Schritt (c) die Aufreinigung und/oder Auftrennung mindestens einer Mischung M unter Erhalt von mindestens einer weiteren Mischung F enthaltend mindestens eine Verbindung ausgewählt aus 2,2'-Methylendiphenyldiisocyanat, 2,4'-Methylendiphenyldiisocyanat und 4,4'-Methylendiphenyldiisocyanat.

Die Aufreinigung und/oder Auftrennung der Mischung oder der Mischungen M enthaltend zweikernige Isomere des Methylendiphenyldiisocyanat ist dem Fachmann an sich bekannt. In Schritt (c) erfolgt vorzugsweise die Auftrennung der Mischung M oder der Mischungen M in mindestens zwei Mischungen F, welche sich in dem Anteil der Isomere 4,4'-MDI, 2,4'-MDI und 2,2'-MDI von der jeweiligen Mischung M unterscheiden.

In einer bevorzugten Ausführungsform wird dabei mindestens eine Mischung F1 erhalten, welche einen höheren Gehalt an 4,4'-MDI aufweist als die Mischung M. Der Gehalt der dabei erhaltenen Mischung F1 an 4,4'-MDI beträgt vorzugsweise mindestens 95 Gew.-%, besonders bevorzugt mindestens 97 Gew.-%, insbesondere mindestens 98 Gew.-% bezogen auf das Gesamtgewicht der Mischung F1.

Im Rahmen dieser bevorzugten Ausführungsform wird außerdem mindestens eine weitere Mischung F2 erhalten, welche einen niedrigeren Gehalt an 4,4'-MDI aufweist als die Mischung M. Der Gehalt der dabei erhaltenen Mischung F2 an 2,4'-MDI beträgt vorzugsweise mindestens 15 Gew.-%, besonders bevorzugt mindestens 20 Gew.-%, insbesondere mindestens 25 Gew.-%, bezogen auf das Gesamtgewicht der Mischung F2. Gleichzeitig beträgt der Gehalt an 4,4'-MDI vorzugsweise mindestens 15 Gew.-%, besonders bevorzugt mindestens 25 Gew.-%, insbesondere mindestens 35 Gew.-%, bezogen auf das Gesamtgewicht der Mischung F2.

Die Aufreinigung und/oder Auftrennung mindestens einer Mischung M unter Erhalt von mindestens einer weiteren Mischung F enthaltend mindestens eine Verbindung ausgewählt aus 2,2'-MDI, 2,4'-MDI und 4,4'-MDI erfolgt vorzugsweise destillativ, inbesondere mittels einer Destillationskolonne (nachfolgend Kolonne K2), wobei optional mindestens eine weitere Destillationskolonne zum Einsatz kommen kann.

Die Mischung M isomerer zweikerniger Methylendiphenyldiisocyanate wird der Kolonne K2 vorzugsweise seitlich zugeführt (sogenannter Feed). Die Kolonne K2 umfasst vorzugsweise Trennelemente, wobei Packungen besonders geeignet sind. Verwendbar sind grundsätzlich aber auch Füllkörper oder Böden. Die Kolonne K2 ist vorzugsweise eine Seitenkolonne. Unter Seitenkolonne ist eine Kolonne zu verstehen, die mindestens einen Sumpfabzug, mindestens einen Seitenabzug und mindestens einen Kopfabzug aufweist.

Je nach Gemischzusammensetzung beträgt die Kopftemperatur der Kolonne K2 bevorzugt 165 bis 200 °C. Der Sumpfdruck beträgt vorzugsweise von 11 bis 20 mbar bei bevorzugten Temperaturen von 210 bis 225 °C. Die Kolonne K2 operiert vorzugsweise bei einem Sumpfdruck von 0,1 bis 50 mbar, bevorzugt von 1 bis 30 mbar, besonders bevorzugt 2 bis 15 mbar und bei einer Sumpftemperatur von 150 bis 250 °C, besonders bevorzugt von 180 bis 240 °C, insbesondere von 200 bis 225°C. Hierdurch wird eine hohe Trennwirkung bei gleichzeitig geringer thermischer Belastung erzielt.

Bei der Destillation des Gemisches isomerer Methylendiphenyldiisocyanate in der Kolonne K2 wird vorzugsweise als Seitenstrom 4,4'-Methylendiphenyldiisocyanat mit einer Isomerenreinheit, d.h. einer Reinheit bezogen auf die drei Isomere 2,2'-MDI, 2,4'-MDI und 4,4'-MDI, von mindestens 97 Gew.-% entnommen (nachfolgend erster Seitenstrom bzw. erster Seitenabzug). Außerdem wird bei der Destillation des Gemisches isomerer Methylendiphenyldiisocyanate in der Kolonne K2 als zweiter Seitenabzug, der sich oberhalb des ersten Seitenabzuges befindet, oder als Kopfstrom ein Gemisch aus 2,4'-Diisocyanatodiphenylmethan und 4,4'-Diisocyanatodiphenylmethan mit einem Gewichtsverhältnis von 85:15 bis 15:85 erhalten. Die Ausführung mit einem zweiten Seitenabzug oberhalb des ersten Seitenabzuges ist bevorzugt, da hierdurch eine hohe Reinheit an den gewünschten zweikernigen Isomeren erhalten wird. Der Kopfstrom der Kolonne K2 umfasst außerdem die mit dem Feed zugeführten Leichtsiederkomponenten, wie z.B. Monochlorbenzol.

Der Strom, der am zweiten Seitenabzug beziehungsweise am Kopf der Kolonne K2 erhalten wird, weist bevorzugt einen Gehalt an 2,4'-MDI von 20 bis 95 Gew.-% und einen Gehalt an 4,4'-MDI von 5 bis 80 Gew.-%, jeweils bezogen auf das Gesamtgewicht der zweikernigen Isomere von Methylendiphenyldiisocyanat, welches 100 Gew.-% ergibt, auf.

Das Kolonnen-Rücklaufverhältnis (Verhältnis zurücklaufender Stoffstrom zu entnommenem Stoffstrom) im Kopf der Kolonne K2 wird insbesondere im Bereich von 10 bis 250 eingestellt, liegt jedoch besonders bevorzugt im Bereich von 60 bis 120, wobei der Destillatstrom 1 bis 5 Gew.-% bezogen auf den Feedstrom beträgt. Der Sumpfstrom beträgt 60 bis 90 Gew.-%, vorzugsweise 75 bis 85 Gew.-% des Feedstromes.

In einer ersten bevorzugten Ausführungsform ist die Kolonne K2 eine Trennwandkolonne. Der Aufbau einer solchen Kolonne K2 ist dem Fachmann an sich bekannt und wird beispielsweise in der EP 1475367 A1 beschrieben, deren Inhalt in vollem Umfang einbezogen wird. Die Trennwandkolonne wird vorzugsweise unter den vorstehend für die Kolonne K2 dargelegten Bedingungen betrieben. Die Mischung M isomerer zweikerniger Methylendiphenyldiisocyanate wird der Trennwandkolonne vorzugsweise seitlich im Bereich der Trennwand zugeführt. Der Bereich der Trennwand befindet sich im mittleren Bereich der Kolonne K2. Die Länge der Trennwand wird abhängig von den Prozessbedingungen und von den Eigenschaften der eingesetzten Stoffaustauschelemente gewählt. Die Trennwand teilt die Kolonne in eine Vorfraktionierungszone und eine Hauptfraktionierungszone. Als Trennelemente sind Packungen besonders geeignet. Verwendbar sind grundsätzlich aber auch Füllkörper oder Böden.

Alternativ kann die destillative Abtrennung der zweikernigen MDI-Isomere auch zweistufig ausgestaltet sein, wobei eine erste Destillationsstufe in einer Destillationskolonne ohne Trennwand, und eine zweite Stufe mit Trennwandkolonne durchgeführt wird oder wobei zwei Trennwandkolonnen Verwendung finden. Entsprechende Verfahren werden in der EP 1475367 A1 in den Absätzen [0024] bis [0031] ausgeführt.

In einer zweiten bevorzugten Ausführungsform ist die Kolonne K2 eine Seitenkolonne ohne Trennwand. Die bevorzugten Parameter der Seitenkolonne wurden bereits vorstehend beschrieben. Dabei werden mittels des Kopfabzuges vorzugsweise 2,2'-MDI und Leichtsieder und mittels des Sumpfabzuges vorzugsweise 4,4'-MDI und Hochsieder abgetrennt, wobei der Strom aus Sumpf- und Kopfabzug wieder in Schritt (b) des erfindungsgemäßen Verfahrens eingesetzt werden kann. Der Seitenkolonne wird vorzugsweise wie oben beschrieben in einem ersten Seitenabzug 4,4'-MDI in einer Reinheit von mindestens 97 Gew.-% bezogen auf das Gesamtgewicht des Stoffstromes entnommen sowie oberhalb des ersten Seitenabzuges in einem zweiten Seitenabzug das oben beschriebene Gemisch aus 4,4'-MDI und 2,4'-MDI.

In einer besonders bevorzugten Ausführungsform wird die oben beschriebene Mischung F1 aus einer weiteren Kolonne K1 erhalten, die der Kolonne K2 nachgeschaltet ist. Schritt (c) wird in dieser besonders bevorzugten Ausführungsform wie folgt durchgeführt.

Ein Gemisch I wird der Kolonne K2 bevorzugt als Seitenstrom entnommen und in gasförmiger Form der Kolonne K1 zugeführt. Das Gemisch I ist 4,4'-MDI mit vorzugsweise mindestens 98 Gew.-%, besonders bevorzugt 98,5 bis 99,0 Gew.-% Reinheit bezogen auf das Gesamtgewicht des Gemisches I.

Bevorzugt wird der Gasstrom bestehend aus dem Gemisch I in der Kolonne K1 mit mindestens einer flüssigen Verbindung A in Kontakt gebracht, welche den gleichen oder einen höheren Siedepunkt als 4,4'-Methylendiphenyldiisocyanat aufweist und welche vorzugsweise einen Gehalt an aromatischen Halogenverbindungen von höchstens 50 ppm aufweist.

Grundsätzlich kommen als flüssige Verbindungen A Verbindungen in Betracht, die entweder inert gegenüber 4,4'-Methylendiphenyldiisocyanat sind oder 4,4'-Methylendiphenyldiisocyanat selbst, wobei jeweils der Gehalt an aromatischen Halogenverbindungen vorzugsweise höchstens 50 ppm beträgt, insbesondere höchstens 40 ppm, besonders bevorzugt höchstens 30 ppm, insbesondere höchstens 20 ppm, ganz besonders bevorzugt höchstens 10 ppm.

Geeignete inerte Verbindungen A sind insbesondere Dibenzylether, Terphyl, höhere Phtalsäureester, Naphthalinderivate. Selbstverständlich können auch Mischungen der vorgenannten inerten Verbindungen in Betracht.

Besonders bevorzugt ist die Verbindung A jedoch 4,4'-Methylendiphenyldiisocyanat. Besonders bevorzugt wird als Verbindung A dabei 4,4'-Methylendiphenyldiisocyanat mit einer Reinheit von mindestens 97 Gew.-%, besonders bevorzugt mindestens 98 Gew.-%, insbesondere mindestens 98,5 Gew.-% verwendet. Grundsätzlich kann jedoch auch eine Mischung aus 4,4'-Methylendiphenyldiisocyanat der genannten Reinheit mit mindestens einer oben genannten inerten Verbindungen verwendet werden.

Besonders bevorzugt ist die Verbindung A 4,4'-Methylendiphenyldiisocyanat, welches gemäß dem erfindungsgemäßen Schritt (d) zurückgeführt wird. Hierdurch wird 4,4'-Methylendiphenyldiisocyanat, welches das erfindungsgemäße Verfahren bereits durchlaufen hat und in geeigneter Weise aufbewahrt werden muss, anteilig erneut dem Prozess zugeführt. Hierdurch können gleichzeitig zwei Vorteile realisiert werden: einerseits wird erfindungsgemäß 4,4'-MDI, das arm an aromatischen Halogenverbindungen ist, zugänglich gemacht und andererseits wird gelagertes 4,4-MDI, das infolge der Lagerung teilweise zu Folgeprodukten abgebaut wurde, wieder rezykliert und gelangt in hochreiner Form in die Lagerung, wodurch die Qualität des gelagerten 4,4'-MDI erfindungsgemäß optimiert werden kann (Vermeidung von Sedimenten und Ablagerungen). Hierbei beträgt das Lager-Rücklaufverhältnis (zurückgeführte Menge an 4,4'-MDI im Verhältnis zu der Lagerung neu zugeführten (hinzugewonnenen) Menge 4,4'-MDI bevorzugt von 0,05 bis 0,4, insbesondere von 0,1 bis 0,3.

Ganz besonders bevorzugt wird 4,4'-Methylendiphenyldiisocyanat aus einer Vorrichtung zur Lagerung von 4,4'-Methylendiphenyldiisocyanat zurückgeführt. Eine Vorrichtung zur Lagerung ist dabei jede Vorrichtung, die zur temporären Aufnahme des zu lagernden Stoffes oder Stoffgemisches vorgesehen ist, beispielsweise ein Behältnis, insbesondere ein Lagertank.

Die Zugabe der flüssigen Verbindung A erfolgt vorzugsweise oberhalb des obersten Trennelementes der Kolonne K1. In einer bevorzugten Ausführungsform ist die Kolonne K1 eine Packungskolonne. Die spezifische Oberfläche der Packung beträgt dabei vorzugsweise von 100 bis 1000 m²/m³, besonders bevorzugt von 150 bis 800 m²/m³, insbesondere von 200 bis 750 m²/m³, ganz besonders bevorzugt von 250 bis 600 m²/m³.

Grundsätzlich sind solche Packungen bevorzugt, die einen geringen Druckverlust verursachen. Geeignete Packungen sind insbesondere Gewebepackungen, Blechpackungen und strukturierte Packungen. Gewebepackungen sind besonders bevorzugt. Die Zugabe der flüssigen Verbindung A erfolgt dabei vorzugsweise oberhalb des höchsten Packungselementes der Kolonne K1.

Grundsätzlich kommen mehrere Methoden des Inkontaktbringens in Betracht. Bevorzugt sind dabei solche Methoden, die zu einem intensiven Kontakt des Gasstromes und der flüssigen Verbindung A führen. Hierfür muss die Flüssigkeit A in geeigneter Weise verteilt werden. Entsprechende Methoden sind dem Fachmann an sich bekannt.

Die Wirksamkeit des Inkontaktbringens ist insbesondere von einer gleichmäßigen, flächendeckenden Flüssigkeitsaufgabe abhängig. Flüssigkeitsverteiler gewährleisten eine weitgehend homogene Flüssigkeitsverteilung über dem Kolonnenquerschnitt und sind dem Fachmann bekannt. Eine Vorverteilung der Flüssigkeit kann durch ein oder mehrere Zulaufrohre oder Verteilerrinnen mit mehrere Ausflussöffnungen, die sich an der Unterseite befinden, realisiert werden.

Eine wichtige Auslegungsgröße ist die Anzahl von Aufgabestellen bezogen auf den Kolonnenquerschnitt (= Tropfstellendichte). Folgende Bauarten von Flüssigkeitsverteilern kommen in Betracht: Verteilerböden, Rinnenverteiler, Rohrverteiler und Düsenverteiler. Folgende Prinzipien der Flüssigkeitsverteilung kommen in Betracht: Stauhöhenverteilung über Bohrungen im Boden von Verteilern oder seitlich gebohrte Aufgaberöhrchen, Überlaufverteilung z.B. über seitliche Schlitze oder Überlauftüllen und Düsen.

Geeignete Flüssigkeitsverteiler sind insbesondere Kastenrinnenverteiler. Es ist vorteilhaft, wenn das Inkontaktbringen im Gegenstrom zum Gasstrom I erfolgt. Hierdurch ergibt sich ein besonders niedriger Gehalt an aromatischen Halogenverbindungen im resultierenden Gemisch.

Der absolute Druck im Kopf der Kolonne K1 beträgt bevorzugt höchstens 50 mbar, besonders bevorzugt von 1 bis 30 mbar, insbesondere von 2 bis 20 mbar. Die Druckdifferenz zwischen Kopf und Sumpf (Druckabfall) der Kolonne K1 beträgt vorzugsweise von 0,5 bis 30 mbar, bevorzugt von 0,5 bis 20 mbar, besonders bevorzugt von 1 bis 10 mbar, insbesondere von 2 bis 5 mbar. Ein geringer Druckabfall bewirkt eine geringere thermische Belastung des Produkts durch niedrigere Sumpftemperaturen.

Die Temperatur im Sumpf der Kolonne K1 beträgt von 140 bis 270°C, bevorzugt von 150 °C bis 240°C, besonders bevorzugt von 170 bis 230°C, insbesondere von 190 bis 230°C, ganz besonders bevorzugt von 200 bis 225 °C. Hierdurch wird die thermische Belastung bei gegebener Effizienz der Aufreinigung minimiert.

Das Rücklaufverhältnis, hier definiert als das Verhältnis von Waschflüssigkeit zu Brüden W:B [w/w] beträgt W:B = 0,01 bis 2,0 bevorzugt W:B= 0,05 bis 0,5, besonders bevorzugt W:B = 0,1 bis 0,3.

Die Kolonne K1 kann aus verschiedenen Materialien gefertigt werden, sofern die verwendeten Materialien gegenüber den eingesetzten Gemischen bei gegebener Temperatur inert sind. Geeignete Materialien sind insbesondere austenische Edelstähle wie 1.4541 oder 1.4571. Auch höherlegierte Werkstoffe, wie der ferritisch/austenitische 1.4462 sind geeignet. Vorzugsweise wird der Werkstoff 1.4541 verwendet.

Am Kopf der Kolonne K1 wird ein Strom O erhalten, der aus dem aufgereinigten Gemisch enthaltend 4,4'-Methylendiphenydiisocyanat besteht und vorzugsweise einen Gehalt an aromatischen Halogenverbindungen von höchstens 50 ppm aufweist. Anschließend wird der Strom O einer Abkühlung unterzogen, indem der Strom O auf eine Temperatur von vorzugsweise 10 bis 100°C, insbesondere 20 bis 80 °C, insbesondere 20 bis 60 °C abgekühlt wird.

Die Abkühlung des Stromes O wird vorzugsweise in höchstens 5 Sekunden von der Temperatur am Kopf der Kolonne K1 auf eine Temperatur im Bereich von 20°C bis 60°C, vorzugsweise von 30°C bis 50°C vorgenommen. Durch die schnelle Abkühlung wird die Bildung von Folgeprodukten, insbesondere die Bildung dimerer Folgeprodukte weiter reduziert.

### Schritt (d)

Erfindungsgemäß erfolgt im Rahmen von Schritt (d) die Lagerung der in Schritt (c) erhaltenen Mischung F oder Mischungen F sowie der in Schritt (b) erhaltenen Mischung P, wobei ein Teil mindestens einer der gelagerten Mischungen F und/oder P mittels Rückführung wieder in mindestens einem der Schritte (b) oder (c) eingesetzt wird.

Die aus der Lagerung in den Prozess zurückgeführte Menge kann grundsätzlich über einen großen Bereich variieren. Bei einer lediglich geringen zurückgeführten Menge in Bezug auf die der Lagerung zugeführten Gesamtmenge des jeweiligen MDI-Gemisches kann die Bildung von Sedimenten eventuell nicht vollständig verhindert werden. Die Rückführung eines hohen Anteils ist andererseits oft nicht ökonomisch.

Vorzugsweise beträgt die aus der Lagerung zurückgeführte Menge der Mischungen F und/oder P bezogen auf die der Lagerung zugeführten Gesamtmenge der Mischungen F und/oder P von 1 bis 30 Gew.-%, vorzugsweise von 5 bis 25 Gew.-%, insbesondere von 10 bis 20 Gew.-%.

Ein Vorteil der Rückführung ist, die Verbindungen, welche Sedimente und/oder Ablagerungen in MDI bilden, insbesondere Uretonimine und Uretdione, zurückzuspalten, ohne einen zusätzlichen Verfahrensschritt zu etablieren.

Entsprechend erfolgt die Rückführung der gelagerten Mischungen F beziehungsweise P vorzugsweise an eine Stelle der Wiedereinspeisung, an der Temperatur und Verweilzeit dergestalt sind, dass die Rückspaltung von Uretdioneinheiten in Isocyanateinheiten erfolgt. Die Rückführung erfolgt bevorzugt dergestalt, dass die Mischung an einer Stelle wieder eingespeist wird, die wie folgt charakterisiert ist: T > 190°C, Verweilzeit bei dieser Temperatur von 5 bis 30 Minuten.

Außerdem erfolgt die Rückführung der gelagerten Mischungen F beziehungsweise P vorzugsweise an eine Stelle der Wiedereinspeisung, an der Temperatur und Verweilzeit dergestalt sind, dass die Rückspaltung von Uretonimineinheiten in Isocyanat- und Carbodiimideinheiten erfolgt. Die Rückführung erfolgt bevorzugt dergestalt, dass die Mischung an einer Stelle wieder eingespeist wird, die wie folgt charakterisiert ist: T > 190°C, Verweilzeit bei dieser Temperatur von 5 bis 30 Minuten.

Die Rückführung der gelagerten Mischungen F und/oder P kann kontinuierlich (ständige Rückführung eines bestimmten Anteils der gelagerten Mischung) oder diskontinuierlich erfolgen.

In einer ersten bevorzugten Ausführungsform erfolgt die Rückführung der gelagerten Mischungen F und/oder P im Rahmen eines kontinuierlichen Verfahrens. Vorzugsweise beträgt die aus der Lagerung kontinuierlich zurückgeführte Menge der Mischungen F und/oder P bezogen auf die der Lagerung zugeführten Gesamtmenge der Mischungen F und/oder P von 1 bis 30 Gew.-%, vorzugsweise von 5 bis 25 Gew.-%, insbesondere von 10 bis 20 Gew.-%.

In einer zweiten bevorzugten Ausführungsform erfolgt die Rückführung der gelagerten Mischungen F und/oder P im Rahmen eines diskontinuierlichen Verfahrens, vorzugsweise bei Erreichen eines Gehaltes von mehr als 0,5 Gew.-% Uretdion, besonders bevorzugt bei Erreichen eines Gehaltes von mehr als 0,3 Gew.-% Uretdion, berechnet als dimere Methylendiphenyldiisocyanat-Einheit bezogen auf das Gesamtgewicht des gelagerten Methylendiphenyldiisocyanat. Vorzugsweise beträgt die aus der Lagerung diskontinuierlich zurückgeführte Menge der Mischungen F und/oder P bezogen auf die der Lagerung zugeführten Gesamtmenge der Mischungen F und/oder P von 1 bis 30 Gew.-%, vorzugsweise von 5 bis 25 Gew.-%, insbesondere von 10 bis 20 Gew.-%.

Die Lagertemperatur steuert die Kinetik der Bildung der Uretdione und Uretonimine. Die Temperatur der gelagerten Mischungen F und/oder P beträgt vorzugsweise von 5°C bis 60°C, besonders bevorzugt von 10 bis 50°C, besonders bevorzugt von 15 bis 45 °C, insbesondere von 20 bis 45°C.

Die Dauer der Lagerung beträgt vorzugsweise mindestens 1 Tag, insbesondere mindestens 2 Tage, bevorzugt mindestens 3 Tage, besonders bevorzugt mindestens 5 Tage. In einigen Fällen beträgt die Dauer der Lagerung sogar mindestens 1 Woche, insbesondere mindestens 2 Wochen. Die Obergrenze der Lagerung ist durch die nicht in vollem Umfang vermeidliche chemische Veränderung der gelagerten Mischung bedingt und beträgt insbesondere höchstens 4 Wochen, bevorzugt höchstens 3 Wochen, insbesondere höchstens 2 Wochen. Der Fachmann wählt die aus der Lagerung zurückgeführte Menge in geeigneter Weise in Verbindung mit den vorgenannten Lagerdauern. Die vorgenannten Lagedauern können vorteilhaft in Kombination mit den ebenfalls vorgenannten Lagertemperaturen realisiert werden.

Der bevorzugte Ort der Wiedereinspeisung beziehungsweise der Verfahrensschritt, in dem die zurückgeführte MDI-Mischung wieder eingesetzt wird, hängt außerdem von der Zusammensetzung ab, d. h. davon, ob es sich um die Mischung P oder um eine Mischung F handelt. Entsprechende bevorzugte Ausführungsformen werden nachfolgend erläutert.

Selbstverständlich können die erhaltenen Mischungen P und F unabhängig voneinander wieder zurückgeführt werden, insbesondere in unterschiedliche Apparaturen eingespeist beziehungsweise in unterschiedlichen Verfahrensschritten wieder eingesetzt werden.

In einer ersten bevorzugten Ausführungsform wird mindestens ein Teil der gelagerten Mischung P mittels Rückführung wieder in Schritt (b) eingesetzt. Vorzugsweise wird mindestens ein Teil der gelagerten Mischung P in eine in Schritt (b) verwendete Trennapparatur, vorzugsweise eine Kolonne oder ein Verdampfer, insbesondere ein Dünnschichtverdampfer oder ein Fallfilmverdampfer, zurückgeführt.

Vorzugsweise wird der zurückgeführte Teil der gelagerten Mischung P in den Umpumpkreislauf eines Fallfilmverdampfers zur Trennung von rohem Methylendiphenyldiisocyanat in die Mischungen M und P zurückgeführt.

In einer weiteren bevorzugten Ausführungsform wird mindestens eine Mischung F1 enthaltend mindestens 95 Gew.-% 4,4'-Methylendiphenyldiisocyanat bezogen auf das Gesamtgewicht der Mischung F1 zurückgeführt und wieder in Schritt (c) eingesetzt. Mischungen F1 wurden bereits weiter oben näher beschrieben.

Vorzugsweise beträgt der Gehalt der zurückgeführten Mischung F1 an 4,4'-Methylendiphenyldiisocyanat mindestens 97 Gew.-% beträgt, vorzugsweise mindestens 98 Gew.-%, insbesondere mindestens 99 Gew.-%, bezogen auf das Gesamtgewicht der zurückgeführten Mischung F1.

Besonders bevorzugt wird mindestens eine Mischung F1 in die oben beschriebene Kolonne K2 zur destillativen Auftrennung der Mischung M zurückgeführt und im Rahmen von Schritt (d) eingesetzt. Die Wiedereinspeisung erfolgt vorzugsweise entweder an der gleichen Stelle wie der Feed oder unterhalb des Feeds.

In einer ganz besonders bevorzugten Ausführungsform wird die oben beschriebene Mischung F1 in eine Kolonne K1 zur destillativen Aufreinigung von 4,4'-Methylendiphenyldiisocyanat zurückgeführt, wobei die beschriebene destillative Aufreinigung von 4,4'-Methylendiphenyldiisocyanat im Anschluss an die destillative Auftrennung der Mischung M mittels der oben beschriebenen Kolonne K2 erfolgt.

Vorzugsweise erfolgt die Rückführung in flüssiger Form oberhalb des obersten Trennelementes der Kolonne K1 als Verbindung A wie weiter oben im Rahmen von Schritt (c) ausgeführt.

In einer weiteren bevorzugten Ausführungsform wird mindestens eine gelagerte Mischung F2 enthaltend 4,4'-Methylendiphenyldiisocyanat und 2,4'-Methylendiphenyldiisocyanat in eine Kolonne K2 zur destillativen Auftrennung einer Mischung M im Rahmen von Schritt (c) zurückgeführt. DieWiedereinspeisung erfolgt vorzugsweise entweder an der gleichen Stelle wie der Feed der Kolonne K2 (Eingangsstrom zweikerniger MDI-Isomere) oder oberhalb des genannten Feeds. Mischungen F1 wurden bereits weiter oben näher beschrieben.

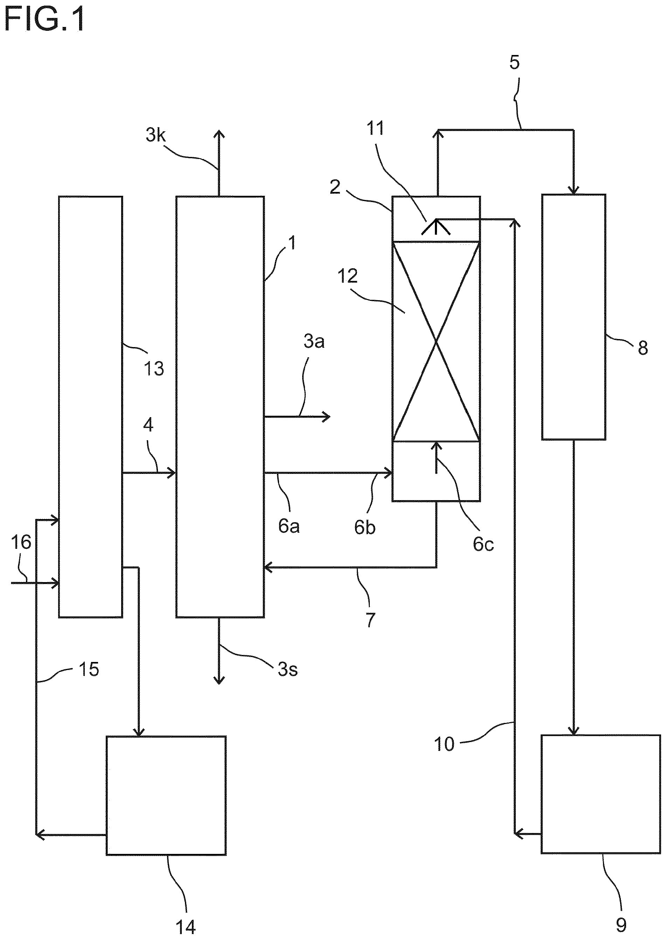

Eine bevorzugte Ausführungsform der vorliegenden Erfindung ist in Fig. 1 schematisch veranschaulicht. Fig. 1 dient dabei der Erläuterung dieser bevorzugten Ausführungsform der vorliegenden Erfindung und ist nicht einschränkend zu verstehen. Einzelne Elemente der nachfolgend erläuterten bevorzugten Ausführungsform können vorteilhaft mit oben erläuterten Ausführungsformen kombiniert werden.

In Fig. 1 bedeutet:
1 - Kolonne K2
2 - Kolonne K1
3a - oberer Seitenabzug der Kolonne K2
3k - Kopfstrom aus der Kolonne K2
3s - Sumpfstrom aus der Kolonne K2
4 - Strom 2-kernige MDI-Isomere (Feed Kolonne K2)
5 - Kopfstrom O der Kolonne K1
6a - unterer Seitenabzug der Kolonne K2
6b - Strom aus Gemisch I
6c - Dampf aus Gemisch I
7 - Sumpfstrom aus Kolonne K1
8 - Kondensator
9 - Lagertank für flüssiges 4,4'-MDI
10 - Rückführung aus Lagertank
11 - Flüssigkeitsverteiler
12 - Packungselemente
13 - Apparatur zur Abtrennung 2-kerniges MDI und PMDI
14 - Lagertank für flüssiges PMDI
15 - Rückführung von PMDI
16 - Feed Roh-MDI

Beschreibung der bevorzugten Ausführungsform gemäß Fig. 1:
Zunächst erfolgt die Zuführung des Feeds an Roh-MDI (16) in eine Apparatur (13) zur Abtrennung der zweikernigen Isomere des MDI einerseits und eine Mischung enthaltend PMDI anderseits. Bei der Apparatur (13) handelt es sich vorzugsweise um einen Fallfilmverdampfer. Das in Anschluss an die Abtrennung ggf. nach Kondensation und/oder Wärmeaustausch erhaltene flüssige PMDI wird einem Lagertank (14) zugeführt. Die erfindungsgemäße Rückführung (15) der gelagerten PMDI-haltigen Mischung in die Apparatur (13) erfolgt bevorzugt oberhalb des Feeds (15). Ein Strom (4) enthaltend eine Mischung zweikerniger Isomere des MDI wird der Kolonne K2 (1) zugeführt. Am Sumpf der Kolonne K2 (1) wird der Sumpfstrom (3s) erhalten und am Kopf der Kopfstrom (3k). Aus einem ersten (6a) wird ein Strom (6b) entnommen, welcher bevorzugt mindestens 98 Gew.-% 4,4'-MDI enthält sowie oberhalb des ersten Seitenstromes ein Strom (3a), welcher aus 4,4'-MDI und 2,4'-MDI besteht. Der Strom (6b) bildet den Strom I, welcher der Kolonne K1 (2) in gasförmigem Zustand zugeführt wird und als Dampf bestehend aus dem Gemisch I (6c) die Packungselemente (12) der Kolonne K1 (2) durchläuft. Im Gegenstrom zur Richtung des Stromes 6c durchläuft 4,4'-MDI, welches einem Lagertank (9) für flüssiges 4,4'-MDI entnommen und über eine Rückführung (10) am Kopf der Kolonne K1 einem Flüssigkeitsverteiler (11) zugeführt wird. Der Flüssigkeitsverteiler (11) sorgt für eine hohe Kontaktfläche zwischen dem aufsteigenden gasförmigen Strom 6c und dem sich im Gegenstrom bewegenden flüssigen Strom aus 4,4'-MDI. Am Sumpf der Kolonne K1 (2) wird der Strom (7) erhalten, welcher in die Kolonne K2 (1) zurückgeführt wird. Am Kopf der Kolonne K1 wird der Kopfstrom O (5) erhalten, welcher aus dem aufgereinigten 4,4'-MDI besteht. Der gasförmige Strom (5) wird einem Kondensator (8) zugeführt, in welchem der Strom (5) kondensiert wird. Anschließend wird das so erhaltene hochreine flüssige 4,4'-MDI einem Lagertank (9) zugeführt.

### Beispiele

### Beispiel 1 (erfindungsgemäß)

Es wurde eine Apparatur verwendet, wie sie in Fig. 1 schematisch dargestellt ist.

2,0 kg/h Roh-MDI enthaltend 50,2 Gew.-% 4,4'-MDI, 6,8 Gew.-% 2,4'-MDI, 21,2 Gew.-% 3-Ring MDI wurden in einem Fallfilmverdampfer aus dem Werkstoff 1.4571 bei einem Druck von 5 mbar verdampft.

Nach der Kondensation wurde ein Destillat enthaltend 85,1 Gew.-% 4,4'-MDI, 12,6 Gew.-% 2,4'-MDI, 2,3 Gew.-% 3-Ring-MDI gewonnen. Der Massenstrom des am Kondensator niedergeschlagenen Destillats betrug 0,690 kg/h.

Das Destillat wurde einer Seitenabzugskolonne K2 in flüssiger Form zugeführt. Die Kolonne K2 und der Sumpfverdampfer bestanden aus dem Werkstoff 1.4571. Die Kolonne war mit strukturierten Packungen ausgestattet, die einen geringen Druckverlust aufwiesen Der Kopfdruck der Kolonne betrug 5 mbar.

Die Kolonne K2 bestand aus einem Verstärkungs- und einem Abtriebsteil. Im Abtriebsteil der Kolonne K2 wurde der Strom I gasförmig mittels eines ersten Seitenabzuges (6a) unterhalb eines Packungselements entnommen und der Kolonne K1 zugeführt (Strom aus Gemisch I). Der Sumpfablauf der Kolonne K1 (7) wurde der Kolonne K2 unterhalb des Zulaufs (Feed) zur Kolonne K2 zugeführt. Oberhalb des Zulaufs wurde an einem oberen Seitenabzug (3a) eine flüssige Fraktion entnommen.

Der Strom I (6b) enthielt 98,7 Gew.-% 4,4'-MDI und 1,3 Gew.-% 2,4'-MDI. Der Gehalt an 3-Ring Verbindungen betrug 530 ppm. Der Massenstrom (6) betrug 0,79 kg/h. Am Strom (3k) wurde eine Mischung von 47 Gew.-% 4,4'-MDI und 53 Gew.-% 2,4'-MDI abgezogen. Der Massenstrom betrug 0,14 kg/h.

Aufreinigung Seitenstrom (6): Der gasförmige Seitenstrom (Dampf aus Gemisch I, 6c) wurde der Kolonne K1 (2) zugeführt. Die Kolonne K1 aus dem Werkstoff 1.4571 war mit einer druckverlustarmen Packung (strukturiert) mit einer spezifischen Oberfläche von 500 m²/m³ ausgestattet. Der Kopfdruck der Kolonne betrug 15 mbar.

Zuführung der flüssigen Verbindung A: Am Kopf der Kolonne K1 wurde ein Strom der Verbindung A bestehend aus 98,5 Gew.-% 4,4'-MDI und 1,5 Gew.-% 2,4'-MDI in flüssiger Form oberhalb des höchsten Packungselements zugegeben und mittels eines Kastenrinnen-Flüssigkeitsverteilers (11) verteilt. Der Massenstrom der Verbindung A betrug 0,17 kg/h. Die Flüssigkeit A bestand aus bei 42°C gelagertem 4,4'-MDI aus einem Lagertank (9), in welchem hochreines 4,4'-MDI aufgenommen wurde, welches das Verfahren schon durchlaufen hat. Der Dimergehalt der Verbindung A betrug 0,13 Gew.-%. Das Gewichtsverhältnis von in Kolonne K1 zurückgeführtem Produkt zu der dem Lagertank zugeführtem Produkt betrug kontinuierlich 0,26. Am Kopf der Kolonne K1 wurde als Produkt ein gasförmiger Strom O (5) erhalten, der aus 98,5 Gew.-% 4,4'-MDI und 1,5 Gew.-% 2,4'-MDI bestand. Der Massenstrom betrug hier 0,65 kg/h. Der Strom O (5) wurde binnen 5s in einem Kondensator (8) auf 42°C abgekühlt und dem Lagertank (9) zugeführt.

## Patentansprüche

1. Verfahren zur Herstellung von Methylendiphenyldiisocyanat umfassend folgende Schritte:
(a) Phosgenierung von zwei- und/oder mehrkernigem Methylendiphenyldiamin unter Erhalt von zwei- und/oder mehrkernigem Methylendiphenyldiisocyanat,
(b) Auftrennung des rohen Methylendiphenyldiisocyanat unter Erhalt von mindestens einer Mischung M enthaltend zweikernige Isomere des Methylendiphenyldiisocyanat und mindestens einer Mischung P enthaltend oligomeres Methylendiphenyldiisocyanat,
(c) Aufreinigung und/oder Auftrennung mindestens einer Mischung M unter Erhalt von mindestens einer weiteren Mischung F enthaltend mindestens eine Verbindung ausgewählt aus 2,2'-Methylendiphenyldiisocyanat, 2,4'-Methylendiphenyldiisocyanat und 4,4'-Methylendiphenyldiisocyanat, und
(d) Lagerung der in Schritt (c) erhaltenen Mischung oder Mischungen F sowie der in Schritt (b) erhaltenen Mischung P für einen Zeitraum von mindestens 1 Tag bei einer Temperatur von 5°C bis 60°C,
**dadurch gekennzeichnet, dass** ein Teil mindestens einer der gelagerten Mischungen F und/oder P mittels Rückführung wieder in mindestens einem der Schritte (b) oder (c) eingesetzt wird, wobei die Rückführung der gelagerten Mischungen F und/oder P an eine Stelle erfolgt, an der Temperatur und Verweilzeit dergestalt sind, dass die Rückspaltung von Uretdioneinheiten in Isocyanateinheiten erfolgt.

2. Verfahren nach Anspruch 1, wobei die aus der Lagerung zurückgeführte Menge der Mischungen F und/oder P bezogen auf die der Lagerung zugeführten Gesamtmenge der Mischungen F und/oder P von 1 bis 30 Gew.-% beträgt, vorzugsweise von 5 bis 25 Gew.-%, insbesondere von 10 bis 20 Gew.-%.

3. Verfahren nach einem oder mehreren der Ansprüche 1 oder 2, wobei die Rückführung der gelagerten Mischungen F oder P an eine Stelle erfolgt, an der Temperatur und Verweilzeit dergestalt ist, dass die Rückspaltung von Uretonimineinheiten in Isocyanat- und Carbodiimideinheiten erfolgt.

4. Verfahren nach einem oder mehreren der Ansprüche 1 bis 3, wobei die Rückführung der gelagerten Mischungen F und/oder P im Rahmen eines kontinuierlichen Verfahrens erfolgt.

5. Verfahren nach einem oder mehreren der Ansprüche 1 bis 4, wobei die Rückführung der gelagerten Mischungen F und/oder P im Rahmen eines diskontinuierlichen Verfahrens erfolgt, vorzugsweise bei Erreichen eines Gehaltes von mindestens 0,3 Gew.-% Uretdion berechnet als dimere Methylendiphenyldiisocyanat-Einheit bezogen auf das Gesamtgewicht des gelagerten Methylendiphenyldiisocyanat.

6. Verfahren nach einem oder mehreren der Ansprüche 1 bis 5, wobei mindestens ein Teil der gelagerten Mischung P mittels Rückführung wieder in Schritt (b) eingesetzt wird.

7. Verfahren nach Anspruch 6, wobei mindestens ein Teil der gelagerten Mischung P in eine in Schritt (b) verwendete Trennapparatur, vorzugsweise ein Kolonne oder ein Verdampfer, insbesondere ein Dünnschichtverdampfer oder ein Fallfilmverdampfer, zurückgeführt wird.

8. Verfahren nach Anspruch 6 oder 7, wobei der zurückgeführte Teil der gelagerten Mischung P in den Umpumpkreislauf eines Fallfilmverdampfers zur Trennung von rohem Methylendiphenyldiisocyanat in die Mischungen M und P zurückgeführt wird.

9. Verfahren nach einem oder mehreren der Ansprüche 1 bis 8, wobei mindestens eine Mischung F1 enthaltend mindestens 95 Gew.-% 4,4'-Methylendiphenyldiisocyanat bezogen auf das Gesamtgewicht der Mischung F zurückgeführt und in Schritt (c) eingesetzt wird.

10. Verfahren nach Anspruch 9, wobei der Gehalt der zurückgeführten Mischung F1 an 4,4'-Methylendiphenyldiisocyanat mindestens 97 Gew.-% beträgt, vorzugsweise mindestens 98 Gew.-%, bezogen auf das Gesamtgewicht der zurückgeführten Mischung F.

11. Verfahren nach Anspruch 9 oder 10, wobei mindestens eine Mischung F1 in mindestens eine Kolonne K2 zur destillativen Auftrennung einer Mischung M zurückgeführt und im Rahmen von Schritt (d) eingesetzt wird.

12. Verfahren nach einem oder mehreren der Ansprüche 9 bis 11, wobei mindestens eine Mischung F1 in eine Kolonne K1 zur destillativen Aufreinigung von 4,4'-Methylendiphenyldiisocyanat zurückgeführt wird, wobei die genannt destillative Aufreinigung von 4,4'-Methylendiphenyldiisocyanat im Anschluss an die destillative Auftrennung der Mischung M mittels mindestens einer Kolonne K2 erfolgt.

13. Verfahren nach Anspruch 12, wobei die Rückführung in flüssiger Form oberhalb des obersten Trennelementes der Kolonne K1 erfolgt.

14. Verfahren nach einem oder mehreren der Ansprüche 9 bis 13, wobei von 1 bis 30 Gew.-% der Mischung F1, bezogen auf das Gesamtgewicht der gelagerten Mischung F, zurückgeführt wird.

15. Verfahren nach einem oder mehreren der Ansprüche 1 bis 14, wobei mindestens eine gelagerte Mischung F2 enthaltend 4,4'-Methylendiphenyldiisocyanat und 2,4'-Methylendiphenyldiisocyanat in eine Kolonne K2 zur destillativen Auftrennung einer Mischung M im Rahmen von Schritt (c) zurückgeführt wird.

## Claims

1. A process for preparing methylenedi(phenyl isocyanate), which comprises the following steps:
(a) phosgenation of two-ring and/or multiring methylenedi(phenylamine) to give two-ring and/or multiring methylenedi(phenyl isocyanate),
(b) fractionation of the crude methylenedi(phenyl isocyanate) to give at least one mixture M comprising two-ring isomers of methylenedi(phenyl isocyanate) and at least one mixture P comprising oligomeric methylenedi(phenyl isocyanate),
(c) purification and/or fractionation of at least one mixture M to give at least one further mixture F comprising at least one compound selected from among 2,2'-methylenedi(phenyl isocyanate), 2,4'-methylenedi(phenyl isocyanate) and 4,4'-methylenedi(phenyl isocyanate), and
(d) storage of the mixture or mixtures F obtained in step (c) and also the mixture P obtained in step (b) for a period of at least 1 day at a temperature from 5°C to 60°C,
wherein part of at least one of the stored mixtures F and/or P is reused by means of recirculation in at least one of the steps (b) or (c), wherein the recirculation of the stored mixtures F and/or P is effected at a point at which the temperature and residence time are such that redissociation of uretdione units into isocyanate units occurs.

2. The process according to claim 1, wherein the amount of the mixtures F and/or P recirculated from storage, based on the total amount of mixtures F and/or P fed to storage, is from 1 to 30% by weight, preferably from 5 to 25% by weight, in particular from 10 to 20% by weight.

3. The process according to one or more of claims 1 and 2, wherein the recirculation of the stored mixtures F or P is effected at a point at which the temperature and residence time are such that redissociation of uretonimine units into isocyanate and carbodiimide units occurs.

4. The process according to one or more of claims 1 to 3, wherein the recirculation of the stored mixtures F and/or P is carried out within a continuous process.

5. The process according to one or more of claims 1 to 4, wherein the recirculation of the stored mixtures F and/or P is carried out within a batch process, preferably when a content of at least 0.3% by weight of uretdione, calculated as dimeric methylenedi(phenyl isocyanate) unit and based on the total weight of the stored methylenedi(phenyl isocyanate), has been reached.

6. The process according to one or more of claims 1 to 5, wherein at least part of the stored mixture P is reused in step (b) by means of recirculation.

7. The process according to claim 6, wherein at least part of the stored mixture P is recirculated to a separation apparatus used in step (b), preferably a column or a vaporizer, in particular a thin film evaporator or a falling film evaporator.

8. The process according to claim 6 or 7, wherein the recirculated part of the stored mixture P is recirculated to the pumped circuit of a falling film evaporator for the separation of crude methylenedi(phenyl isocyanate) into the mixtures M and P.

9. The process according to one or more of claims 1 to 8, wherein at least one mixture F1 comprising at least 95% by weight of 4,4'-methylenedi(phenyl isocyanate), based on the total weight of the mixture F, is recirculated and used in step (c).

10. The process according to claim 9, wherein the 4,4'-methylenedi(phenyl isocyanate) content of the recirculated mixture F1 is at least 97% by weight, preferably at least 98% by weight, based on the total weight of the recirculated mixture F.

11. The process according to claim 9 or 10, wherein at least one mixture F1 is recirculated to at least one column K2 for the distillative fractionation of a mixture M and is used in step (d).

12. The process according to one or more of claims 9 to 11, wherein at least one mixture F1 is recirculated to a column K1 for the distillative purification of 4,4'-methylenedi(phenyl isocyanate), with said distillative purification of 4,4'-methylenedi(phenyl isocyanate) being carried out after the distillative fractionation of the mixture M by means of at least one column K2.

13. The process according to claim 12, wherein the recirculation is effected in liquid form above the uppermost separation element of the column K1.

14. The process according to one or more of claims 9 to 13, wherein from 1 to 30% by weight of the mixture F1, based on the total weight of the stored mixture F, is recirculated.

15. The process according to one or more of claims 1 to 14, wherein at least one stored mixture F2 comprising 4,4'-methylenedi(phenyl isocyanate) and 2,4'-methylenedi(phenyl isocyanate) is recirculated to a column K2 for the distillative fractionation of a mixture M in step (c).

## Revendications

1. Procédé de fabrication de diisocyanate de diphénylméthylène comprenant les étapes suivantes :
(a) la phosgénation de méthylène-diphényldiamine bi-et/ou polynucléaire pour obtenir du diisocyanate de diphénylméthylène bi- et/ou polynucléaire,
(b) la séparation du diisocyanate de diphénylméthylène brut pour obtenir au moins un mélange M contenant des isomères binucléaires du diisocyanate de diphénylméthylène et au moins un mélange P contenant du diisocyanate de diphénylméthylène oligomère,
(c) la purification et/ou la séparation d'au moins un mélange M pour obtenir au moins un autre mélange F contenant au moins un composé choisi parmi le diisocyanate de 2,2'-diphénylméthylène, le diisocyanate de 2,4'-diphénylméthylène et le diisocyanate de 4,4'-diphénylméthylène, et
(d) l'entreposage du mélange ou des mélanges F obtenus à l'étape (c), ainsi que du mélange P obtenu à l'étape (b) pendant une durée d'au moins 1 jour à une température de 5 °C à 60 °C,
**caractérisé en ce qu'**une partie d'au moins un des mélanges F et/ou P entreposés est réutilisée par recyclage dans au moins une des étapes (b) et/ou (c), le recyclage des mélanges F et/ou P entreposés ayant lieu à un emplacement auquel la température et le temps de séjour sont tels que le rétroclivage d'unités uretdione en unités isocyanate a lieu.

2. Procédé selon la revendication 1, dans lequel la quantité des mélanges F et/ou P recyclée à partir de l'entreposage, par rapport à la quantité totale des mélanges F et/ou P introduits dans l'entreposage, est de 1 à 30 % en poids, de préférence de 5 à 25 % en poids, notamment de 10 à 20 % en poids.

3. Procédé selon une ou plusieurs des revendications 1 ou 2, dans lequel le recyclage des mélanges F ou P entreposés a lieu à un emplacement auquel la température et le temps de séjour sont tels que le rétroclivage d'unités urétonimine en unités isocyanate et carbodiimide a lieu.

4. Procédé selon une ou plusieurs des revendications 1 à 3, dans lequel le recyclage des mélanges F et/ou P entreposés a lieu dans le cadre d'un procédé continu.

5. Procédé selon une ou plusieurs des revendications 1 à 4, dans lequel le recyclage des mélanges F et/ou P entreposés a lieu dans le cadre d'un procédé discontinu, de préférence lorsqu'une teneur d'au moins 0,3 % en poids d'uretdione, calculée en tant qu'unité diisocyanate de diphénylméthylène dimère, par rapport au poids total du diisocyanate de diphénylméthylène entreposé, est atteinte.

6. Procédé selon une ou plusieurs des revendications 1 à 5, dans lequel au moins une partie du mélange P entreposé est réutilisée dans l'étape (b) par recyclage.

7. Procédé selon la revendication 6, dans lequel au moins une partie du mélange P entreposé est recyclée dans un appareil de séparation utilisé à l'étape (b), de préférence une colonne ou un évaporateur, notamment un évaporateur à couche mince ou un évaporateur à film tombant.

8. Procédé selon la revendication 6 ou 7, dans lequel la partie recyclée du mélange P entreposé est recyclée dans le circuit de pompage d'un évaporateur à film tombant pour la séparation de diisocyanate de diphénylméthylène brut en les mélanges M et P.

9. Procédé selon une ou plusieurs des revendications 1 à 8, dans lequel au moins un mélange F1 contenant au moins 95 % en poids de diisocyanate de 4,4"-diphénylméthylène, par rapport au poids total du mélange F, est recyclé et utilisé à l'étape (c).

10. Procédé selon la revendication 9, dans lequel la teneur du mélange F1 recyclé en diisocyanate de 4,4'-diphénylméthylène est d'au moins 97 % en poids, de préférence d'au moins 98 % en poids, par rapport au poids total du mélange F recyclé.

11. Procédé selon la revendication 9 ou 10, dans lequel au moins un mélange F1 est recyclé dans au moins une colonne K2 pour la séparation par distillation d'un mélange M, et utilisé dans le cadre de l'étape (d).

12. Procédé selon une ou plusieurs des revendications 9 à 11, dans lequel au moins un mélange F1 est recyclé dans une colonne K1 pour la purification par distillation de diisocyanate de 4,4'-diphénylméthylène, ladite purification par distillation de diisocyanate de 4,4'-diphénylméthylène ayant lieu après la séparation par distillation du mélange M au moyen d'au moins une colonne K2.

13. Procédé selon la revendication 12, dans lequel le recyclage a lieu sous forme liquide au-dessus de l'élément de séparation le plus supérieur de la colonne K1.

14. Procédé selon une ou plusieurs des revendications 9 à 13, dans lequel de 1 à 30 % en poids du mélange F1, par rapport au poids total du mélange F entreposé, est recyclé.

15. Procédé selon une ou plusieurs des revendications 1 à 14, dans lequel au moins un mélange F2 entreposé contenant du diisocyanate de 4,4'-diphénylméthylène et du diisocyanate de 2,4'-diphénylméthylène est recyclé dans une colonne K2 pour la séparation par distillation d'un mélange M dans le cadre de l'étape (c).
